# FASCICULE DE BREVET EUROPEEN

(11) **EP 1 474 046 B1**
(45) Date de publication et mention de la délivrance du brevet: **26.03.2008**
(21) Numéro de dépôt: 03712296.7
(22) Date de dépôt: 11.02.2003
(51) Int. Cl.: A61B 17/16

(54) **DISPOSITIF PERMETTANT LE SUIVI DE PENETRATION DANS DES ELEMENTS ANATOMIQUES**
VORRICHTUNG ZUM ERMITTELN DES DURCHDRINGENS IM KÖRPER
DEVICE FOR MONITORING PENETRATION INTO ANATOMICAL MEMBERS

(30) Priorité: 11.02.2002 FR 0201652
(43) Date de publication de la demande: 10.11.2004
(73) Titulaire: Spinevision, 75012 Paris (FR)
(72) Inventeur: BOURLION, Maurice, F-42400 Saint-Chamond (FR); VANQUAETHEM, Alain, F-13840 Rognes (FR)
(74) Mandataire: Breese, Pierre
(86) Numéro de dépôt international: PCT/FR2003/000440
(87) Numéro de publication internationale: WO 2003/068076

(56) Documents cités:
- WO-A-01/01875
- DE-A- 19 758 110
- FR-A- 2 101 911
- FR-A- 2 691 623
- GB-A- 2 335 990
- US-A- 4 243 388
- US-A- 4 595 019
- US-A- 4 630 615
- US-A- 5 941 876

## Description

La présente invention se rapporte au domaine de la pénétration des structures osseuses, présentant des différences de grandeurs physiques intrinsèques.
La présente invention se rapporte plus particulièrement à un dispositif permettant le suivi de la pénétration d'un moyen de pénétration dans des structures osseuses d'un corps vivant, lesdites structures présentant au moins deux zones d'impédances électriques différentes.

En chirurgie du rachis, par exemple, lors du forage pédiculaire, il est fréquent que le cortex osseux vertébral soit traversé, cassé ou ébréché par l'outil de pénétration. Selon les auteurs, 15 à 40% des vis pédiculaires seraient ainsi mal positionnées, avec une traduction clinique dans 1 à 2% des cas (douleur, paralysie, hémorragie ...) et donc la nécessité d'intervenir à nouveau.
Les chirurgiens utilisent parfois des équipements tels que des équipements de :
- navigation chirurgicale, coûteuse et lourde de mise en oeuvre,
- surveillance des potentiels évoqués sensitifs et/ou moteurs, moins coûteuse mais également contraignante, car nécessitant la présence d'un spécialiste dont la mission est uniquement d'opérer cette surveillance.
Il en résulte que dans la majorité des cas, les opérateurs comptent uniquement sur leurs connaissances de l'anatomie et leur expérience pour accomplir ce geste chirurgical à risques.

Ce qui précède est également vrai pour d'autres domaines de la chirurgie.

Dans le domaine du perçage des corps osseux, l'art antérieur connaît également la demande internationale de brevet N° WO 01/01875 . Le préambule de la revendication 1 est basé sur ce document.
Cette demande de brevet présente un dispositif qui utilise la capacité des nerfs et des muscles à transmettre des signaux, pour alarmer l'utilisateur lorsque le moyen de perçage entre en contact avec un nerf, afin d'empêcher toute lésion dudit nerf.

L'art antérieur connaît également l'usage de la mesure d'impédance dans des dispositifs médicaux.
L'art antérieur connaît, par exemple, le brevet américain N° US 4 630 615 qui porte sur un système de stimulation neuronale incorporant un appareil de mesure ou de détermination d'impédance dans lequel il est souhaité de gérer et déterminer les changements d'impédance dans un guide connecté à une cathode implantée dans l'espace épidural d'une colonne vertébrale. Un tel système de stimulation neuronale est typiquement utilisé lorsque l'on souhaite bloquer des signaux de douleur qui vont vers le cerveau et peut aussi être utilisé pour le traitement et/ou pour l'allègement des symptômes de mouvements de la colonne vertébrale désordonnés tels que l'épilepsie, la spasticté, la paralysie cérébrale, etc.

L'art antérieur connaît également la demande de brevet britannique N° GB 2 335 990 qui porte sur un système permettant de faire pénétrer mécaniquement une aiguille et de stopper la pénétration si une impédance différente est constatée à l'extrémité de l'aiguille.
Le but de ce système est d'informer lorsque l'extrémité de l'aiguille a atteint d'une manière certaine la profondeur souhaitée, soit en utilisant une valeur d'impédance, soit en utilisant une variation d'impédance.
Le but de ce système n'est donc absolument pas d'empêcher d'atteindre une profondeur déterminée.
En outre, ce système impose une détermination expérimentale pour chaque patient des valeurs ou des variations d'impédance, comme exposé en dernière page de cette demande, qui ne permet absolument pas d'adapter ce système dans un but d'empêcher d'atteindre une profondeur déterminée.

L'art antérieur connaît également la demande de brevet français N° FR 2 101 911 qui porte sur un instrument de mesure de la longueur du canal d'une racine de dent.
Cet instrument permet de déduire la longueur d'un corps creux par la mesure postérieure de la distance entre deux repères après que ces deux repères aient été mis en butés longitudinale, respectivement dans le fond du canal de la dent pour la sonde, et la couronne ou le bord supérieur de la cavité pour le morceaux de caoutchouc.
Cet instrument permet d'être informé, après avoir réalisé une cavité, de la profondeur de cette cavité ; il ne permet pas un suivi en temps réel de la pénétration d'un moyen de pénétration dans des structures anatomiques.

Un autre inconvénient des techniques de l'art antérieur est qu'elles sont lourdes à mettre en oeuvre et tendent à allonger le temps de l'opération, augmentant ainsi les risques associés.

La présente invention entend remédier aux inconvénients de l'art antérieur en proposant un dispositif qui permette de suivre en temps réel la pénétration du moyen de pénétration (moyen de perçage ou autre) dans le matériau, en mesurant les différences de grandeur physique au fur et à mesure de la pénétration, afin de permettre à l'utilisateur de savoir où se trouve l'extrémité du moyen de pénétration et empêcher ainsi toute lésion.
La grandeur physique utilisée est la mesure d'impédance électrique.
Il est important de remarquer que, dans des réalisations qui n'appartiennent pas à la présente invention, l'utilisation de la mesure d'impédance électrique peut être appliquée à beaucoup de domaines techniques différents, et pas seulement le domaine du perçage des corps osseux.

La présente invention se rapporte, dans son acception la plus large, à un dispositif permettant le suivi de la pénétration d'un moyen de pénétration selon la revendication 1.
Ce dispositif comporte au moins :
■ au moins un impédancemètre pouvant être relié à au moins deux électrodes dont l'une au moins est situé à une extrémité distale dudit moyen de pénétration, ledit impédancemètre mesurant en permanence l'impédance entre les deux électrodes au moins pendant la pénétration, et
■ au moins un dispositif d'alerte capable de produire un signal d'alerte en cas de détection, par l'impédancemètre d'une variation d'impédance.
Dans une variante, le dispositif comporte en outre un électrostimulateur apte à réaliser une stimulation et pouvant être relié à au moins deux électrodes dont l'une au moins est situé à une extrémité distale dudit moyen de pénétration,
Dans cette variante, au moins une électrode pouvant être reliée à un électrostimulateur et au moins une électrode pouvant être reliée à un impédancemètre sont de préférence reliées entre elles. Ainsi, ce sont les mêmes électrodes qui servent à l'envoi de la stimulation neuromusculaire et à la mesure d'impédance.

Dans tout le texte qui suit, l'extrémité distale s'entend bien sûr, comme pour tout instrument chirurgical, par rapport au manche qui permet de se saisir de l'instrument, situé à l'extrémité proximale.
Ledit signal d'alerte est de préférence un signal visuel et/ou sonore et/ou tactile.
La stimulation neuromusculaire réalisée par l'électrostimulateur neuromusculaire présente de préférence une fréquence inférieure ou égale à 10Hz, une tension inférieure ou égale à 4 Volts et une impulsion de durée inférieure ou égale à 400 µS.

Dans une variante de réalisation, une électrode est constituée par une surface de contact située à l'extrémité distale dudit moyen de pénétration, et une autre électrode est constituée par une surface de contact, destinée à être positionnée sur une surface extérieure des structures anatomiques.
Dans une autre variante de réalisation, lesdites électrodes sont constituées chacune respectivement par une surface de contact située à l'extrémité distale dudit moyen de pénétration, lesdites surfaces de contact étant séparées par un isolant.
Dans une version de cette variante, l'électrode proximale présente une surface de contact supérieure à la surface de contact de l'électrode distale.
Dans une variante de réalisation, au moins une électrode distale est constituée par une surface de contact située sur une partie périphérique partielle de l'extrémité distale du moyen de pénétration, de manière à permettre de détecter une variation d'impédance dans une direction sensiblement perpendiculaire à l'axe de pénétration du moyen de pénétration.

Le dispositif selon l'invention comporte de préférence des moyens d'entraînement en rotation dudit moyen de pénétration.
Dans une version de l'invention, , l'impédancemètre, le dispositif d'alerte et éventuellement l'électrostimulateur sont positionnés sur une carte électronique amovible, comportant des moyens pour connecter lesdites électrodes, afin de permettre de stériliser le dispositif indépendamment de la partie électronique.
Le dispositif selon l'invention comporte à cet effet, de préférence, un manche creux pour l'accueil de ladite carte électronique.

Le dispositif selon la présente invention peut comprendre une carte électronique telle que définie ci-dessus.
Cette carte électronique est, de préférence, stérilisable et est, de préférence également, positionnée dans une enveloppe préservant la stérilité.
La présente invention se rapporte à un instrument de forage manuel ou motorisé, notamment pour le forage du pédicule vertébral, ledit instrument comportant un moyen de pénétration et un dispositif permettant le suivi de la pénétration dudit moyen de pénétration.

Avantageusement, la présente invention permet d'être informé en temps réel de la progression du moyen de pénétration associé éventuellement à l'instrument de forage.
Avantageusement également, l'électrode située en amont de la pénétration est positionnée au près de l'effet mécanique de pénétration et permet ainsi d'obtenir une très grande sensibilité de détection de variation d'impédance.
Avantageusement, l'invention permet d'éviter ainsi de traverser complètement le corps à pénétrer ou à percer lorsque cela n'est pas souhaité et, dans le cas de la pénétration de corps osseux, de provoquer une lésion des tissus situés sous le corps osseux.

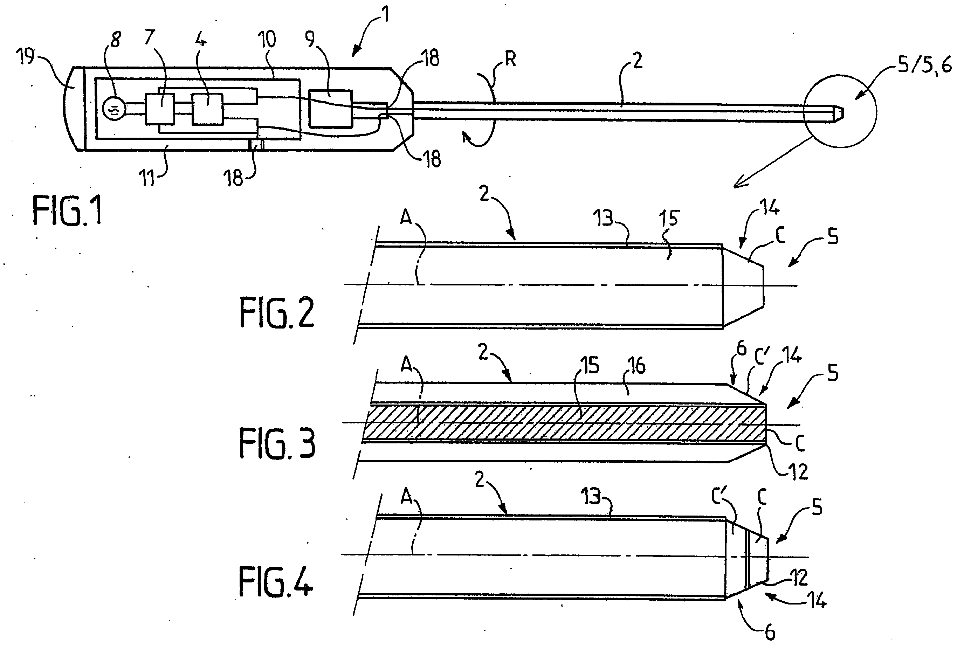

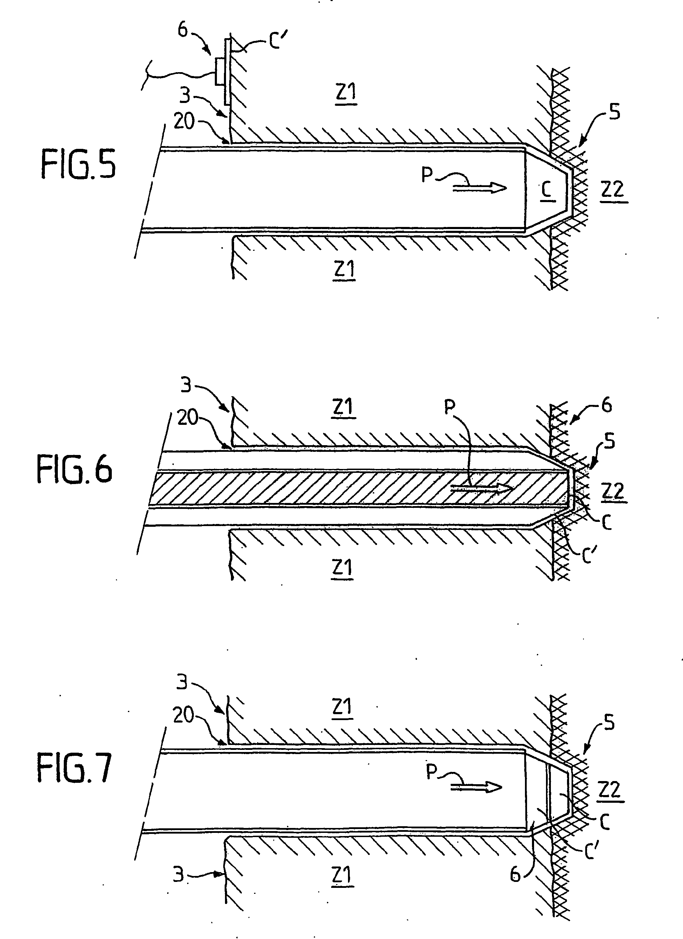

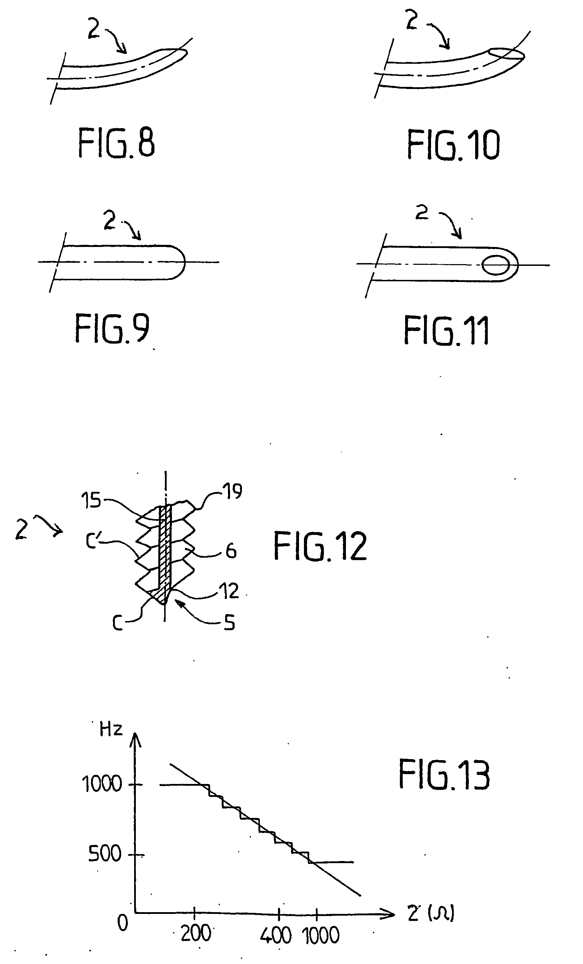

On comprendra mieux l'invention à l'aide de la description, faite ci-après à titre purement explicatif, d'un mode de réalisation de l'invention, en référence aux figures annexées :
■ la figure 1 illustre une vue en coupe partielle d'un instrument équipé d'un dispositif selon l'invention ;
■ la figure 2 illustre une vue en coupe en détail d'une première variante de réalisation du moyen de pénétration en forme de sonde ;
■ la figure 3 illustre une vue en coupe en détail d'une version de la deuxième variante de réalisation du moyen de pénétration en forme de sonde ;
■ la figure 4 illustre une vue en coupe en détail d'une autre version de la deuxième variante de réalisation du moyen de pénétration en forme de sonde ;
■ la figure 5 illustre une vue en coupe de la pénétration P du moyen de pénétration de la figure 2 ;
■ la figure 6 illustre une vue en coupe de la pénétration P du moyen de pénétration de la figure 3 ;
■ la figure 7 illustre une vue en coupe de la pénétration P du moyen de pénétration de la figure 4 ;
■ les figures 8 et 9 illustrent respectivement une vue de face et une vue de dessus d'un moyen de pénétration selon l'invention constitué par une spatule ;
■ les figures 10 et 11 illustrent respectivement une vue de face et une vue de dessus d'un moyen de pénétration selon l'invention constitué par une curette ;
■ la figure 12 illustre une vue en coupe en détail d'un moyen de pénétration selon l'invention constitué par une mèche, une vis ou un taraud ; et
■ la figure 13 illustre deux exemples de relations entre l'impédance mesurée Z en ohm et la fréquence en hertz d'un signal sonore émis par le dispositif d'alerte.

Le dispositif (1) selon l'invention, illustré figure 1, est un dispositif permettant le suivi de la pénétration d'un moyen de pénétration (2) dans des structures osseuses (3) d'un corps vivant, lesdites structures présentant au moins deux zones d'impédance électrique différentes (Z1, Z2).
Le moyen de pénétration (2) permet, lui-même ou en association avec un instrument de forage, de forer un trou (20) dans lesdites structures osseuses (3).
Un corps osseux est composé de tissus mous internes (moelle, spongieux) et durs externes (cortex) ; il est lui-même environné de tissus mous : muscles, tendons et ligaments, vaisseaux et nerfs.
De par ces différences de nature, le cortex osseux présente des caractéristiques physiques différentes de celles des tissus avec lesquels il est en contact : c'est d'ailleurs pour cela qu'il est visible sur des images médicales : radiographie classique, scanner, échographie, Imagerie par Résonance Magnétique.
Si l'instrument avec lequel on pénètre dans le cortex osseux est équipé à son extrémité distale d'un moyen détectant et signalant cette différence de caractéristiques physiques, l'opérateur disposera alors immédiatement d'une information lui indiquant qu'il a ou qu'il va traverser ce cortex.
Or, il se trouve que le cortex osseux et les tissus mous présentent également des impédances différentes.

Dans une version de base, le dispositif (1) selon l'invention comporte :
■ au moins un impédancemètre (7) pouvant également être relié à au moins deux électrodes dont l'une au moins est situé à une extrémité distale dudit moyen de pénétration (2), ledit impédancemètre mesurant en permanence l'impédance entre les deux électrodes au moins pendant la pénétration, et
■ au moins un dispositif d'alerte (8) capable de produire un signal d'alerte en cas de détection, par l'impédancemètre (7) d'une variation d'impédance.

Ainsi, grâce à l'impédancemètre (7), il est possible de suivre la pénétration du moyen de détection associé au moyen de pénétration (2) au fur et à mesure qu'il pénètre dans les structures osseuses (3).

La manière dont on utilise le ou les impédancemètres (7) pour en déduire la position du moyen de détection associé constitue un procédé particulier de suivi de la pénétration d'un moyen de pénétration (2) au travers d'un corps présentant au moins deux zones d'impédance électrique différentes (Z1, Z2).

Le dispositif (1) comporte un dispositif d'alerte (8) qui peut émettre :
■ un signal d'alerte visuel modulé, par exemple par la mise en oeuvre d'un témoin lumineux clignotant ou d'une rampe de témoins lumineux et/ou
■ un signal d'alerte sonore modulé en fréquence et éventuellement en intensité, par exemple par la mise en oeuvre d'un haut parleur, et/ou
■ un signal d'alerte tactile modulé en fréquence et éventuellement en intensité, par exemple par la mise en oeuvre d'un vibreur,
permettant d'avertir, dès le début de la variation de l'impédance mesurée, et lors du franchissement d'un seuil d'une variation de l'impédance mesurée.
Ce signal d'alerte modulé est proportionnel à la variation d'impédance.
Ce dispositif d'alerte (8) est de préférence positionné sur ou dans l'instrument, comme visible sur la figure 1.

Le dispositif (1) peut éventuellement en outre comporter un moyen de visualisation de la ou des variation(s) de l'impédance, relié à un impédancemètre (7). Ce moyen de visualisation, constitué par exemple d'un écran de visualisation, permet de suivre l'évolution, sous forme de courbes, de la variation de l'impédance au fur et à mesure de la pénétration du moyen de pénétration (2).

Dans une variante, le dispositif (1) comporte en outre au moins un électrostimulateur (4), de préférence neuromusculaire, apte à réaliser une stimulation de préférence neuromusculaire et pouvant être relié à au moins deux électrodes (5, 6) dont l'une au moins est situé à une extrémité distale dudit moyen de pénétration (2).
La stimulation neuromusculaire réalisée par l'électrostimulateur neuromusculaire présente :
■ une fréquence inférieure ou égale à 10Hz, de préférence de l'ordre de 2,5 Hz,
■ une tension inférieure ou égale à 4 Volts, de préférence de l'ordre de 1 Volts, et
■ une impulsion de durée inférieure ou égale à 400 µS, de préférence de l'ordre de 150 µS.
En cas de franchissement du cortex osseux par le moyen de pénétration, la partie distale (pointe) de ce dernier vient au contact des tissus mous situés à l'extérieur de l'enveloppe corticale. Les impulsions de stimulation peuvent alors se propager facilement dans ces tissus mous de faible impédance et stimuler les nerfs se trouvant éventuellement à proximité de la pointe du moyen de pénétration. Deux cas se présentent alors :
a) s'il s'agit de nerfs moteurs, ils vont alors commander des contractions des groupes musculaires qui leurs sont associés, à la cadence des impulsions de stimulation ; ces contractions seront détectées et reconnues, soit par un électromyographe préalablement connecté au patient, soit cliniquement par l'opérateur lui-même par rapport aux mouvements du patient.
b) s'il s'agit de nerfs sensitifs, un dispositif adapté pourra détecter leur stimulation, également reconnaissable par son rythme.

Dans une version préférée de l'invention, les électrodes (5, 6) peuvent être reliées, grâce à des bornes de branchement (18), à la fois à l'électrostimulateur (4) et à l'impédancemètre (7). Ainsi, ce sont les mêmes électrodes qui servent à la fois pour l'émission de la stimulation neuromusculaire et pour la mesure d'impédance.

Le moyen de pénétration (2) du dispositif (1) peut être :
■ fixe, et constituer notamment une sonde, une pointe carrée, une spatule ou une curette, ... ou
■ susceptible d'être entraîné en rotation, et constituer notamment une vis, un taraud ou une mèche de forage.
Dans ce dernier cas, le dispositif (1) comporte alors des moyens d'entraînement (9) en rotation R dudit moyen de pénétration (2).
Les moyens d'entraînement (9) sont constitués, par exemple d'un moteur électrique capable d'entraîner le moyen de pénétration (2) en rotation et forment en association avec le moyen de pénétration (2) un instrument (1) de perçage du type perceuse.

Dans la partie ci-après, le moyen de pénétration (2) est constitué par une sonde, mais les configurations présentées sont applicables à tout type de moyen de pénétration (2).
Dans une première variante, illustrée figures 2 et 5, une électrode (5) distale est constituée par une surface de contact C située à l'extrémité distale dudit moyen de pénétration (2), et une autre électrode (6) proximale est constituée par une surface de contact C', destinée à être positionnée sur une surface extérieure des structures anatomiques, y compris la plaie opératoire.
Le moyen de pénétration (2) comporte une partie centrale (15) conductrice et une partie périphérique (13) isolée jusqu'à une extrémité distale (14) mise à nu sur quelques millimètres, c'est-à-dire non isolée. La surface C présente une taille inférieure à 10 mm², de l'ordre de 4 mm² et la surface C' présente une taille de l'ordre de 20 mm².
Ainsi, le premier pôle du dispositif électronique de stimulation et de mesure est formé par l'extrémité distale du moyen de pénétration (2) de l'instrument et l'autre pôle est formé par une connexion de référence au patient.

Dans une deuxième variante, illustrée figures 3 et 6 d'une part et 4 et 7 d'autre part, lesdites électrodes (5, 6) sont constituées chacune respectivement par une surface de contact C, C' située à l'extrémité distale dudit moyen de pénétration (2), lesdites surfaces de contact C, C' étant séparées par un isolant (12) d'épaisseur inférieure ou égale à 1 mm.

Dans la version illustrée figures 3 et 6, le moyen de pénétration (2) comporte une partie centrale (15) conductrice et une partie extérieure (16) conductrice, lesdites partie centrale (15) et partie extérieure (16) étant séparées par un isolant (12) cylindrique. Les deux parties conductrices forment chacune un pôle du dispositif électronique.
Ainsi, la partie extérieure (16) forme un tube extérieur conducteur, évidé en son centre et la partie centrale (15) forme un cylindre intérieur conducteur, les deux parties centrale (15) et extérieure (16) débouchant à l'extrémité du moyen de pénétration (2), afin de former les deux surfaces C et C', isolés l'une par rapport à l'autre.
Dans cette version, l'électrode (6) proximale présente une surface de contact C' supérieure à la surface de contact C de l'électrode (5) distale. La surface C présente une taille inférieure à 10 mm², de l'ordre de 4 mm² et la surface C' présente une taille supérieure à 100 mm², de l'ordre de 400 mm².

Dans la version illustrée figures 4 et 7, le moyen de pénétration (2) comporte une partie périphérique (13) isolante et une extrémité distale (14), non isolée, présentant deux électrodes (5, 6) positionnées perpendiculairement à l'axe A dudit moyen de pénétration (2) et séparées par un isolant (12). Les deux électrodes (5, 6) forment chacune un pôle du dispositif électronique.
Dans cette version, les électrodes (5, 6) présentent des surfaces de contact, respectivement C et C' de tailles sensiblement identiques, inférieures à 10 mm² et de l'ordre de 4 mm². Les surfaces de contact C, C' sont séparées par un isolant (12) d'épaisseur inférieure ou égale à 1 mm selon un axe A de pénétration dudit moyen de pénétration (2).

Les figures 5, 6 et 7 illustrent un moment au cours de la pénétration P du moyen de pénétration (2) au cours duquel une variation d'une grandeur physique et plus particulièrement une variation d'impédance électrique, est détectée par l'impédancemètre (7). Une telle variation se produit notamment lorsque le moyen de pénétration (2) quitte le cortex du corps osseux, représenté par la zone Z1 et pénètre dans un tissus environnant mou représenté par la zone Z2.
Dans la variante associant un impédancemètre et un électrostimulateur, les impulsions de stimulation du dispositif ont une forme, une tension et une intensité connues ; il est donc possible d'en déduire l'impédance mesurées par les électrodes associées au dispositif et en particulier par les électrodes placées à la partie distale (pointe) du moyen de pénétration.

Cette information "impédance locale" peut alors être transformée en signaux capables de mettre en oeuvre le dispositif d'alerte (8).

Le moyen de pénétration (2) peut comporter une, voire plusieurs paire(s) d'électrodes.
Pour chaque paire d'électrodes, au moins une électrode est positionnée à l'extrémité distale (14) dudit moyen de pénétration (2).

Dans une variante de réalisation, au moins une électrode (5) distale est constituée par une surface de contact C située sur une partie périphérique partielle de l'extrémité distale du moyen de pénétration (2), de manière à permettre de détecter une variation d'impédance dans une direction sensiblement perpendiculaire à l'axe A de pénétration du moyen de pénétration (2). Ainsi, en tournant le moyen de pénétration (2) et en observant le dispositif d'alerte (8), il est possible de déduire de la variation d'impédance mesurée grâce à cette électrode, la configuration de l'extrémité distale du trou (20).

Dans la partie ci-après, le moyen de pénétration (2) est constitué par une mèche.
Le moyen de pénétration (2) est muni, par exemple d'un filet hélicoïdal (19), représenté figure 12, ou de plusieurs filets hélicoïdaux, formé(s) selon l'axe A, afin de permettre de permettre de forer des trous (20).
La mèche comporte une partie centrale (15) conductrice et un filet hélicoïdal (19') conducteur, la partie centrale (15) et le filet hélicoïdal (19) étant séparées par un isolant (12) cylindrique. Les deux parties conductrices forment chacune un pôle du dispositif électronique.

Dans cette version, l'électrode (6) proximale présente une surface de contact C' supérieure à la surface de contact C de l'électrode (5) distale. La surface C présente une surface inférieure à 10 mm², de l'ordre de 4 mm² et la surface C' présente une surface supérieure à 10 mm², de l'ordre de 40 mm². Les surfaces de contact C, C' sont séparées d'un isolant (12) d'épaisseur inférieure ou égale à 1 mm selon un axe A de pénétration dudit moyen de pénétration (2).
Cette configuration est également applicable à une vis et notamment une vis autoforeuse.

Dans une variante de l'invention, l'impédancemètre (7) et le dispositif d'alerte (8) ou l'impédancemètre (7), le dispositif d'alerte (8) et l'électrostimulateur (4), sont positionnés sur une carte électronique (10) amovible, visible sur la figure 1, comportant des moyens pour connecter lesdites électrodes (5, 6). Ainsi, la partie mécanique du dispositif (1) peut être stérilisée en autoclave, sans la partie électronique.
Il est également possible de prévoir que la carte électronique (10) est à usage unique : elle est livrée dans un emballage approprié, est associée par le chirurgien avec l'instrument dont il a besoin (sonde, pointe carrée, spatule, curette, tournevis, perceuse, ...) lorsque le chirurgien souhaite opérer un contrôle de la pénétration au fur et à mesure qu'il se sert de l'instrument associé. La carte électronique (10) est jetée après l'opération, alors que l'instrument est stérilisé.

Il est également possible de réaliser une carte électronique (10) qui puisse être stérilisée, notamment par action chimique.

La carte électronique (10) peut par ailleurs être positionnée dans une enveloppe préservant la stérilité.
Pour permettre l'association de la carte électronique (10) avec l'instrument, le dispositif (1) comporte de préférence un manche (11) creux dans lequel la carte électronique (10) peut être positionnée. L'accès à l'intérieur du manche creux se fait en enlevant un bouchon (19).
Les bornes de branchement (18) permettent de relier les électrodes. Elles peuvent être positionnées en périphérie du manche (11), pour permettre de brancher une ou plusieurs électrodes, et dans la partie distale du manche par rapport au bouchon (19), pour permettre de relier la ou les électrode(s) située(s) à l'extrémité distale du moyen de pénétration (2).

Le dispositif électronique de stimulation et de mesure positionné sur la carte électronique (10) mesure, pendant l'utilisation de l'instrument, l'impédance électrique entre les deux pôles constitués chacun par une électrode et signale en temps réel (de façon sonore, visuelle ou tactile) les valeurs et/ou variations de ladite impédance, en particulier en cas d'effraction du cortex pédiculaire.
Sur la figure 13, on peut observer deux représentation de relations entre l'impédance mesurée, en abscisse et la fréquence d'un signal sonore émis par le dispositif d'alerte, en ordonnée. Cette relation peut être par exemple linéaire, illustrée en trait discontinu, ou par palier. Cette relation est obtenue avec des surfaces C et C' de l'ordre de 5 mm².

Pour la mise en oeuvre du dispositif selon l'invention à un procédé de perçage, il peut être utile de prévoir un ensemble, ou jeu, de moyens de pénétration (2) dont chaque moyen de pénétration (2) présente un diamètre différent.

L'invention est décrite dans ce qui précède à titre d'exemple. Il est entendu que l'homme du métier est à même de réaliser différentes variantes de l'invention sans pour autant sortir du cadre du brevet qui est défini par les revendications.

## Revendications

1. Dispositif (1) de forage permettant le suivi de la pénétration d'un moyen de pénétration (2) dans une structure osseuse (3) d'un corps vivant, ladite structure présentant au moins deux zones d'impédances électriques différentes (Z1, Z2), **caractérisé en ce qu'**il comporte au moins un impédancemètre (7) pouvant être relié à au moins deux électrodes (5, 6) dont l'une au moins est située à une extrémité distale dudit moyen de pénétration (2), ledit impédancemètre mesurant en permanence l'impédance entre les deux électrodes (5, 6) au moins pendant la pénétration et au moins un dispositif d'alerte (8) délivrant un signal d'alerte modulé proportionnellement à la variation d'impédance détectée par l'impédancemètre (7).

2. Dispositif (1) de forage selon la revendication 1, **caractérisé en ce que** ledit signal d'alerte est un signal d'alerte visuel modulé.

3. Dispositif (1) de forage selon la revendication 1, **caractérisé en ce que** ledit signal d'alerte est un signal d'alerte sonore modulé en fréquence.

4. Dispositif (1) de forage selon la revendication 1 ou 3, **caractérisé en ce que** ledit signal d'alerte est un signal d'alerte sonore modulé en intensité.

5. Dispositif (1) de forage selon la revendication 1, **caractérisé en ce que** ledit signal d'alerte est un signal d'alerte tactile modulé en fréquence.

6. Dispositif (1) de forage selon la revendication 1 ou 5, **caractérisé en ce que** ledit signal d'alerte est un signal d'alerte tactile modulé en intensité.

7. Dispositif (1) de forage selon la revendication 3 ou 4, **caractérisé en ce que** la fréquence d'un signal sonore émis par le dispositif d'alerte est une fonction linéaire de l'impédance mesurée.

8. Dispositif (1) de forage selon la revendication 3 ou 4, **caractérisé en ce que** la fréquence d'un signal sonore émis par le dispositif d'alerte est une fonction par pallier de l'impédance mesurée.

9. Dispositif (1) de forage selon l'une quelconque des revendications précédentes, **caractérisé en ce qu'**il comporte au moins un électrostimulateur (4) apte à réaliser une stimulation et pouvant être relié à au moins deux électrodes (5, 6) dont l'une au moins est située à une extrémité distale dudit moyen de pénétration (2).

10. Dispositif (1) de forage selon la revendication 9, **caractérisé en ce qu'**au moins une électrode (5, 6) pouvant être reliée à un électrostimulateur (4) et au moins une électrode (5, 6) pouvant être reliée à un impédancemètre (7) sont reliées entre elles.

11. Dispositif (1) de forage selon la revendication 9 ou 10, **caractérisé en ce que** la stimulation neuromusculaire réalisée par l'électrostimulateur (4) présente une fréquence inférieure ou égale à 10Hz, une tension inférieure ou égale à 4 Volts et une impulsion de durée inférieure ou égale à 400 µS.

12. Dispositif (1) de forage selon l'une quelconque des revendications 1 à 11, **caractérisé en ce qu'**une électrode (5) est constituée par une surface de contact C située à l'extrémité distale dudit moyen de pénétration (2), et **en ce qu'**une autre électrode (6) est constituée par une surface de contact C', destinée à être positionnée sur une surface extérieure des structures anatomiques.

13. Dispositif (1) de forage selon l'une quelconque des revendications 1 à 11, **caractérisé en ce que** lesdites électrodes (5, 6) sont constituées chacune respectivement par une surface de contact C, C' située à l'extrémité distale dudit moyen de pénétration (2), lesdites surfaces de contact C, C' étant séparées par un isolant (12).

14. Dispositif (1) de forage selon la revendication 4, **caractérisé en ce que** l'électrode (6) proximale présente une surface de contact C' supérieure à la surface de contact C de l'électrode (5) distale.

15. Dispositif (1) de forage selon l'une quelconque des revendications précédentes, **caractérisé en ce que** le moyen de pénétration (2) comporte une partie centrale (15) conductrice et une partie extérieure (16) conductrice, lesdites partie centrale (15) et partie extérieure (16) étant séparées par un isolant (12) cylindrique, la partie extérieure (16) formant un tube extérieur conducteur, évidé en son centre et la partie centrale (15) formant un cylindre intérieur conducteur, les deux parties centrale (15) et extérieure (16) débouchant à l'extrémité du moyen de pénétration (2), afin de former deux surfaces C et C', isolés l'une par rapport à l'autre.

16. Dispositif (1) de forage selon l'une quelconque des revendications précédentes, **caractérisé en ce que** l'électrode (6) proximale présente une surface de contact C' supérieure à la surface de contact C de l'électrode (5) distale et **en ce que** la surface C présente une surface inférieure à 10 mm², de l'ordre de 4 mm² et la surface C' présente une surface supérieure à 10 mm², de l'ordre de 40 mm², les surfaces de contact C, C' étant séparées d'un isolant (12) d'épaisseur inférieure ou égale à 1 mm selon un axe A de pénétration dudit moyen de pénétration (2).

17. Dispositif (1) de forage selon l'une quelconque des revendications précédentes, **caractérisé en ce qu'**au moins une électrode (5) distale est constituée par une surface de contact C située sur une partie périphérique partielle de l'extrémité distale du moyen de pénétration (2), de manière à permettre de détecter une variation d'impédance dans une direction sensiblement perpendiculaire à l'axe A de pénétration du moyen de pénétration (2).

18. Dispositif (1) de forage selon l'une quelconque des revendications 1 à 17, **caractérisé en ce qu'**il comporte des moyens d'entraînement en rotation (9) dudit moyen de pénétration (2).

19. Dispositif (1) de forage selon l'une quelconque des revendications 1 à 18, **caractérisé en ce que** l'impédancemètre (7), le dispositif d'alerte (8) et éventuellement l'électrostimulateur (4) sont positionnés sur une carte électronique (10) amovible, comportant des moyens pour connecter lesdites électrodes (5, 6).

20. Dispositif (1) de forage selon la revendication 19, **caractérisé en ce qu'**il comporte un manche (11) creux pour l'accueil de ladite carte électronique (10).

21. Dispositif (1) de forage selon la revendication 19 ou la revendication 20, **caractérisée en ce que** la carte électronique (10) est stérilisable.

22. Dispositif (1) de forage selon l'une quelconque des revendications 19 à 21, **caractérisé en ce que** la carte électronique (10) est positionnée dans une enveloppe préservant la stérilité.

## Claims

1. A drilling device (1), making it possible to follow up the penetration of a penetration mean (2) into a bone structure (3) of a living body, said structure having at least two different electric impedance areas (Z1, Z2, **characterized in that** it includes at least one impedance meter (7) which can be connected to at least two electrodes (5, 6), one of which at least is located at a distal end of said penetration mean (2), said impedance meter permanently measuring the impedance between both electrodes (5, 6) at least during the penetration, and at least a warning device (8) delivering a warning signal modulated proportionately to the variation of impedance detected by the impedance meter (7).

2. A drilling device (1) according to claim 1, **characterized in that** said warning signal is a modulated visual warning signal.

3. A drilling device (1) according to claim 1, **characterized in that** said warning signal is a frequency-modulated sound warning signal.

4. A drilling device (1) according to claim 1 or 3, **characterized in that** said warning signal is an intensity-modulated sound warning signal.

5. A drilling device (1) according to claim 1, **characterized in that** said warning signal is a frequency-modulated tactile warning signal.

6. A drilling device (1) according to claim 1 or 5, **characterized in that** said warning signal is an intensity-modulated tactile warning signal.

7. A drilling device (1) according to claim 3 or 4, **characterized in that** the frequency of a sound signal emitted by the warning device is a linear function of the measured impedance.

8. A drilling device (1) according to claim 3 or 4, **characterized in that** the frequency of a sound signal emitted by the warning signal is a step function of the measured impedance.

9. A drilling device (1) according to any one of the preceding claims, **characterized in that** it includes at least one electrostimulator (4) capable of making a stimulation and being able to be connected to at least two electrodes (5, 6), one at least of which is located at a distal end of said penetration mean (2).

10. A drilling device (1) according to claim 9, **characterized in that** at least one electrode (5, 6) which can be connected to an electrode stimulator (4) and at least one electrode (5, 6) which can be connected to an impedance meter (7) are connected together.

11. A drilling device (1) according to claim 9 or 10, **characterized in that** the neuromuscular stimulation performed by the electrostimulator (4) has a frequency smaller than or equal to 10Hz, a voltage smaller than or equal to 4 Volts and a pulse smaller than or equal to 400 microseconds.

12. A drilling device (1) according to any one of claims 1 to 11, **characterized in that** one electrode (5) is composed of a contact surface C located at the distal end of said penetration mean (2), and **in that** another electrode (6) is composed of a contact surface C', intended to be positioned on an external surface of the anatomic structures.

13. A drilling device (1) according to anyone of claims 1 to 11, **characterized in that** said electrodes (5, 6) are composed, each, respectively, of a contact surface C, C' located at the distal end of said penetration mean (2), said contact surfaces C, C' being separated by an insulator (12).

14. A drilling device (1) according to claim 4, **characterized in that** the proximal electrode (6) has a contact surface C' larger than the contact surface C of the distal electrode (5).

15. A drilling device (1) according to anyone of the preceding claims, **characterized in that** the penetration mean (2) includes a conductive central part (15) and a conductive external part (16), said central part (15) and external part (16) being separated by a cylindrical insulator (12), the external part (16) forming a conductive external tube, with a hollow center and the central part (15) forming a conductive internal cylinder, both central (15) and external (16) parts opening at the end of the penetration mean (2), in order to form two surfaces C and C' which are insulated from each other.

16. A drilling device (1) according to any one of the preceding claims, **characterized in that** the proximal electrode (6) has a contact surface C' larger than the contact surface C of the distal electrode (5), and **in that** the surface C has a surface smaller than 10 mm², of the order of 4 mm² and the surface C' has a surface larger than 10 mm², of the order of 40 mm², the contact surfaces C, C' being separated by an insulator (12) having a thickness smaller than or equal to 1 mm, along a penetration axis A of said penetration means (2).

17. A drilling device (1) according to any one of the preceding claims, **characterized in that** at least a distal electrode (5) is composed of a contact surface C located on a partial peripheral part of the distal end of the penetration mean (2), so as to make it possible to detect a variation of impedance in a direction substantially perpendicular to the penetration axis A of the penetration mean (2).

18. A drilling device (1) according to any one of claims 1 to 17, **characterized in that** it includes means (9) for driving into rotation said penetration means (2).

19. A drilling device (1) according to any one of claims 1 to 18, **characterized in that** the impedance meter (7), the warning device (8) and eventually the electrostimulator (4) are positioned on a removable electronic card (10) comprising means for connecting said electrodes (5, 6).

20. A drilling device (1) according to claim 19, **characterized in that** it includes a hollow handle (11) for receiving said electronic card (10).

21. A drilling device (1) according to claim 19 or claim 20, **characterized in that** the electronic card (10) can be sterilized.

22. A drilling device (1) according to any one of claims 19 to 21, **characterized in that** the electronic card (10) is positioned in a sterile pack.

## Patentansprüche

1. Bohrvorrichtung (1) für die Verfolgung der Eindringung eines Penetrationsmittels (2) in eine Knochenstruktur (3) eines lebendigen Körpers, wobei die besagte Struktur mindestens zwei verschiedene elektrische Impedanzzonen (Z1, Z2) aufweist, **dadurch gekennzeichnet, daß** sie mindestens einen Scheinwiderstandsmesser (7) umfaßt, der an mindestens zwei Elektroden (5, 6) angeschlossen werden kann, von denen sich mindestens eine an einem entfernten Ende des besagten Penetrationsmittels (2) befindet, wobei der besagte Scheinwiderstandsmesser zumindest während der Eindringung ständig die Impedanz zwischen den beiden Elektroden (5, 6) mißt, und mindestens eine Warnvorrichtung (8), die ein Warnsignal abgibt, das im Verhältnis zur vom Scheinwiderstandsmesser (7) entdeckten Impedanzschwankung moduliert ist.

2. Bohrvorrichtung (1) nach Anspruch 1, **dadurch gekennzeichnet, daß** das besagte Warnsignal ein moduliertes Sichtwarnsignal ist.

3. Bohrvorrichtung (1) nach Anspruch 1, **dadurch gekennzeichnet, daß** das besagte Warnsignal ein frequenzmoduliertes Tonwarnsignal ist.

4. Bohrvorrichtung (1) nach Anspruch 1 oder 3, **dadurch gekennzeichnet, daß** das besagte Warnsignal ein stärkemoduliertes Tonwarnsignal ist.

5. Bohrvorrichtung (1) nach Anspruch 1, **dadurch gekennzeichnet, daß** das besagte Warnsignal ein frequenzmoduliertes Tastwarnsignal ist.

6. Bohrvorrichtung (1) nach Anspruch 1 oder 5, **dadurch gekennzeichnet, daß** das besagte Warnsignal ein stärkemoduliertes Tastwarnsignal ist.

7. Bohrvorrichtung (1) nach Anspruch 3 oder 4, **dadurch gekennzeichnet, daß** die Frequenz eines von der Warnvorrichtung abgegebenen Tonsignals eine lineare Funktion der gemessenen Impedanz ist.

8. Bohrvorrichtung (1) nach Anspruch 3 oder 4, **dadurch gekennzeichnet, daß** die Frequenz eines von der Warnvorrichtung abgegebenen Tonsignals eine stufenweise Funktion der gemessenen Impedanz ist.

9. Bohrvorrichtung (1) nach einem beliebigen der vorstehenden Ansprüche, **dadurch gekennzeichnet, daß** sie mindestens einen Elektrostimulator (4) umfaßt, der geeignet ist, eine Stimulation auszuführen, und der an mindestens zwei Elektroden (5, 6) angeschlossen werden kann, von denen sich mindestens eine an einem entfernten Ende des besagten Penetrationsmittels (2) befindet.

10. Bohrvorrichtung (1) nach Anspruch 9, **dadurch gekennzeichnet, daß** mindestens eine Elektrode (5, 6), die mit einem Elektrostimulator (4) verbunden werden kann und mindestens eine Elektrode (5, 6), die mit einem Scheinwiderstandsmesser (7) verbunden werden kann, untereinander verbunden sind.

11. Bohrvorrichtung (1) nach Anspruch 9 oder 10, **dadurch gekennzeichnet, daß** die vom Elektrostimulator (4) ausgeführte nervenmuskulare Anregung eine Frequenz von unter oder gleich 10 Hz aufweist, eine Spannung von unter oder gleich 4 Volt und eine Impulsion mit einer Dauer von unter oder gleich 400 µS.

12. Bohrvorrichtung (1) nach einem beliebigen der vorstehenden Ansprüche 1 bis 11, **dadurch gekennzeichnet, daß** eine Elektrode (5) aus einer Kontaktfläche C gebildet wird, die sich am entfernten Ende des besagten Penetrationsmittels (2) befindet, und **dadurch**, daß eine andere Elektrode (6) aus einer Kontaktfläche C' gebildet wird, die auf einer Außenfläche der anatomischen Strukturen positioniert werden soll.

13. Bohrvorrichtung (1) nach einem beliebigen der vorstehenden Ansprüche 1 bis 11, **dadurch gekennzeichnet, daß** die besagten Elektroden (5, 6) jeweils aus einer Kontaktfläche C beziehungsweise C' gebildet werden, die sich am entfernten Ende des besagten Penetrationsmittels (2) befindet, wobei die besagten Kontaktflächen C, C' durch ein Isoliermittel (12) getrennt sind.

14. Bohrvorrichtung (1) nach Anspruch 4, **dadurch gekennzeichnet, daß** die in der Nähe liegende Elektrode (6) eine Kontaktfläche C' aufweist, die größer als die Kontaktfläche C der entfernten Elektrode (5) ist.

15. Bohrvorrichtung (1) nach einem beliebigen der vorstehenden Ansprüche, **dadurch gekennzeichnet, daß** das Penetrationsmittel (2) einen zentralen leitenden Teil (15) und einen äußeren leitenden Teil (16) umfaßt, wobei der besagte zentrale (15) und der besagte äußere (16) Teil durch ein zylinderförmiges Isoliermittel (12) getrennt sind, wobei der äußere Teil (16) ein äußeres leitendes Rohr bildet, das in seiner Mitte ausgenommen ist, und der zentrale Teil (15) einen inneren leitenden Zylinder bildet, wobei die beiden Teile, der zentrale (15) und der äußere (16) zum Ende des Penetrationsmittels (2) führen, und dort zwei Flächen, C und C', bilden, die voneinander getrennt sind.

16. Bohrvorrichtung (1) nach einem beliebigen der vorstehenden Ansprüche, **dadurch gekennzeichnet, daß** die in der Nähe liegende Elektrode (6) eine Kontaktfläche C' aufweist, die größer als die Kontaktfläche C der entfernten Elektrode (5) ist, und **dadurch**, daß die Fläche C eine Fläche unter 10 mm² , nämlich von 4 mm² aufweist, und die Fläche C' eine Fläche von über 10 mm², nämlich von 40 mm² aufweist, wobei die Kontaktflächen C, C' durch ein Isoliermittel (12) getrennt sind, das eine Dicke von unter oder gleich 1 mm auf einer Achse A der Penetration des besagten Penetrationsmittels (2) aufweist.

17. Bohrvorrichtung (1) nach einem beliebigen der vorstehenden Ansprüche, **dadurch gekennzeichnet, daß** mindestens eine entfernte Elektrode (5) aus einer Kontaktfläche C gebildet wird, die sich auf einem teilweise peripheren Teil des entfernten Endes des Eindringungsmittels (2) befindet, so daß eine Variation der Impedanz in einer deutlich senkrecht zur Achse A der Penetration des Penetrationsmittels (2) verlaufenden Richtung entdeckt werden kann.

18. Bohrvorrichtung (1) nach einem beliebigen der vorstehenden Ansprüche 1 bis 17, **dadurch gekennzeichnet, daß** sie Mittel für den Drehantrieb (9) des besagten Penetrationsmittels (2) umfaßt.

19. Bohrvorrichtung (1) nach einem beliebigen der vorstehenden Ansprüche 1 bis 18, **dadurch gekennzeichnet, daß** sich der Scheinwiderstandsmesser (7), die Warnvorrichtung (8) und eventuell der Elektrostimulator (4) auf einer entfernbaren elektronischen Karte (10) befinden, die Mittel für den Anschluß der besagten Elektroden (5, 6) umfaßt.

20. Bohrvorrichtung (1) nach Anspruch 19, **dadurch gekennzeichnet, daß** sie eine Hohlmuffe (11) für die Aufnahme der besagten Elektronikkarte (10) umfaßt.

21. Bohrvorrichtung (1) nach Anspruch 19 oder nach Anspruch 20, **dadurch gekennzeichnet, daß** die Elektronikkarte (10) sterilisierbar ist.

22. Bohrvorrichtung (1) nach einem beliebigen der vorstehenden Ansprüche 19 bis 21, **dadurch gekennzeichnet, daß** die Elektronikkarte (10) sich in einer Hülle befindet, die die Sterilität erhält.
